(19) Europäisches Patentamt
European Patent Office
Office européen des brevets

(11) **EP 3 404 435 B1**

(12) **EUROPÄISCHE PATENTSCHRIFT**

(45) Veröffentlichungstag und Bekanntmachung des Hinweises auf die Patenterteilung:
**31.08.2022 Patentblatt 2022/35**

(21) Anmeldenummer: **17171672.3**

(22) Anmeldetag: **18.05.2017**

(51) Internationale Patentklassifikation (IPC):
**G01R 33/385** (2006.01)    **G01R 33/421** (2006.01)

(52) Gemeinsame Patentklassifikation (CPC):
**G01R 33/3856; G01R 33/385;** G01R 33/4215

(54) **GRADIENTENSPULENEINHEIT FÜR EIN MAGNETRESONANZGERÄT**

GRADIENT COIL UNIT FOR A MAGNETIC RESONANCE DEVICE

UNITÉ DE BOBINES À GRADIENT POUR APPAREIL À RÉSONANCE MAGNÉTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**21.11.2018 Patentblatt 2018/47**

(73) Patentinhaber: **Siemens Healthcare GmbH**
**91052 Erlangen (DE)**

(72) Erfinder: **Kimmlingen, Ralph**
**90513 Zirndorf (DE)**

(56) Entgegenhaltungen:
**EP-A1- 0 587 423      JP-A- 2011 010 760**
**US-A- 5 334 937      US-A1- 2003 197 507**
**US-A1- 2005 179 434      US-A1- 2016 091 576**

**Beschreibung**

Die Erfindung betrifft eine Gradientenspuleneinheit umfassend eine Primärspule mit einer ersten Primärspulenwicklung und einer zweiten Primärspulenwicklung, welche gemeinsam dazu ausgebildet sind, einen Magnetfeldgradient in eine erste Richtung zu erzeugen.

In einem Magnetresonanzgerät wird üblicherweise der zu untersuchende Körper eines Untersuchungsobjektes, insbesondere eines Patienten, mit Hilfe eines Hauptmagneten einem relativ hohen Hauptmagnetfeld, beispielsweise von 1,5 oder 3 Tesla, ausgesetzt. Im Rahmen einer Magnetresonanzbildgebung (MR-Bildgebung) werden mit Hilfe einer Gradientenspuleneinheit Gradientenpulse ausgespielt. Zusätzlich werden über eine Hochfrequenzantenneneinheit dann mittels geeigneter Antenneneinrichtungen hochfrequente Hochfrequenz-Pulse (HF-Pulse), insbesondere Anregungspulse, ausgesendet, was dazu führt, dass die Kernspins bestimmter, durch diese HF-Pulse resonant angeregter Atome um einen definierten Flipwinkel gegenüber den Magnetfeldlinien des Hauptmagnetfelds verkippt werden. Bei der Relaxation der Kernspins werden Hochfrequenz-Signale, so genannte Magnetresonanz-Signale, abgestrahlt, die mittels geeigneter Hochfrequenzantennen empfangen und dann weiterverarbeitet werden. Aus den so akquirierten Rohdaten können schließlich die gewünschten Bilddaten rekonstruiert werden.

Eine Gradientenspuleneinheit umfasst typischerweise drei Primärspulen und drei dazu korrespondierende Sekundärspulen. Eine Primärspule ist typischerweise zur Erzeugung eines Magnetfeldgradienten in eine Raumrichtung ausgelegt. Eine dazu korrespondierende Sekundärspule schirmt den von der Primärspule erzeugten Magnetfeldgradienten derart ab, dass dieser außerhalb der Gradientenspuleneinheit kompensiert wird und/oder außerhalb der Gradientenspuleneinheit kein Magnetfeldgradient entsteht. Die Primärspulen und die Sekundärspulen werden mit elektrischen Strömen gesteuert, deren Amplituden bis zu 1 kA erreichen und die häufigen und raschen Wechseln der Stromrichtung mit Anstiegs- und Abfallraten von mehreren 100 kA/s unterliegen. Die treibende Spannung für den Spulenstrom beträgt bis zu mehreren kV.

Typischerweise ist eine Gradientenspuleneinheit charakterisiert über ihre maximal erzielbare Gradientenamplitude, gegeben durch einen Quotienten aus einer magnetischen Flussdichte und einer Länge. Ein weiteres Charakteristikum für deren Leistung ist die Slewrate. Die Slewrate ist definiert als Quotient aus der maximalen Gradientenamplitude und der Dauer, die benötigt wird, um die maximale Gradientenamplitude zu erreichen. Die Slewrate ist somit ein Maß für die Schnelligkeit von Gradientenspuleneinheiten. Die maximal erzielbare Gradientenamplitude und die Slewrate definieren das maximale Gradientenmoment, das innerhalb einer Zeitdauer von der Gradientenspuleneinheit erzeugt werden kann. Das Gradientenmoment ist proportional zum Zeitintegral der zeitabhängigen Gradientenamplitude. Des Weiteren ist eine Gradientenspuleneinheit durch ihre nominale Gradientenamplitude charakterisiert. Die nominale Gradientenamplitude ist die maximale, im thermischen Gleichgewicht der Gradientenspuleneinheit unter Berücksichtigung ihrer Kühlung zu erzeugende Gradientenamplitude. Die Slewrate einer Gradientenspuleneinheit für ein Ganzkörpersystem ist typischerweise durch Stimulation der peripheren Nerven des Patienten auf etwa 150 /m/s bis 200 T/m/s begrenzt. Die nominale Gradientenamplitude ist typischerweise durch die maximal verfügbare Kühlleistung limitiert, mittels der im Betrieb der Gradientenspuleneinheit entstehende Wärme abgeleitet wird. Die nominale Gradientenamplitude eines klinischen Ganzkörpersystems ist typischerweise auf 20 mT/m bis 40 mT/m begrenzt. Dadurch ist auch das pro Zeiteinheit erzeugbare Gradientenmoment limitiert. Neuentwicklungen im Bereich Gradienten-Echo basierter Neuroapplikationen sind typischerweise durch die pro Zeiteinheit erzeugbaren Gradientenmomente limitiert. Bisherige Weiterentwicklungen für Gradientenspuleneinheiten betreffen hauptsächlich die Kühlung der Gradientenspuleneinheiten, wie beispielsweise DE 10 2013 208 631 B3 und/oder DE 10 2011 083 204 A1.

US 2005/179434 A1 offenbart Spulenanordnungen für Magnetresonanzbildgebung und Spektroskopie. US 2003/197507 A1 offenbart ein Gradientensystem für eine flexible Anpassung des Sichtfeldes. EP 0 587 423 A1 offenbart eine Integration von Gradientenspulen mit dem Hauptmagneten eines Magnetresonanzgerätes. US 2016/091576 A1 offenbart ein Magnetresonanzgerät mit einer HF-Abschirmung. JP 2011 010760 A offenbart eine Kühlung für eine Gradientenspuleneinheit. Aus dem US-Patent 5 334 937 ist ein Gradientensystem bekannt, in dem die Gradientenspulen aus einer Vielzahl von Leitern bestehen, die parallel zueinander in Schichten angeordnet sind. Einzelne Leiter können elektrisch parallel verschaltet sein, um gewünschte Schaltgeschwindigkeiten oder Amplituden zu ermöglichen.

Der Erfindung liegt die Aufgabe zugrunde, ein Gradientensystem anzugeben, welches dazu ausgebildet ist, ein besonders starkes Gradientenmoment pro Zeiteinheit besonders effizient zu erzeugen. Die Aufgabe wird durch die Merkmale der unabhängigen Ansprüche gelöst. Vorteilhafte Ausgestaltungen sind in den Unteransprüchen beschrieben.

Das erfindungsgemäße Gradientensystem umfasst eine erste Spannungsquelle und eine Gradientenspuleneinheit umfassend eine erste Primärspule und eine erste Sekundärspule, wobei die erste Primärspule eine erste Primärspulenwicklung und eine zweite Primärspulenwicklung umfasst. Die erste Primärspulenwicklung und die zweite Primärspulenwicklung sind mit der ersten Spannungsquelle verschaltet und gemeinsam dazu ausgebildet, einen Magnetfeldgradient in eine erste Richtung zu erzeugen, sofern die erste Spannungsquelle einen ersten Strom induziert, wobei die erste Primärspulenwicklung und die zweite Primärspulenwicklung parallel zu der ersten Spannungsquelle geschalten sind.

Die erste Primärspule ist demnach dazu ausgebildet, einen Magnetfeldgradient in eine erste Richtung zu erzeugen, wenn ein erster Strom in die erste Primärspulenwicklung und die zweite Primärspulenwicklung geleitet wird. Eine Primärspulenwicklung umfasst typischerweise eine oder mehrere sattelförmig angeordnete Wicklungen eines Leiters. Eine Primärspulenwicklung kann auch eine spiralförmige Leiteranordnung umfassen. Die erste Primärspulenwicklung und die zweite Primärspulenwicklung sind erfindungsgemäß auf einer Mantelfläche eines Kreiszylinders angeordnet. Eine Spulenwicklung im Allgemeinen ist typischerweise auf einer Mantelfläche eines Kreiszylinders angeordnet. Weitere Geometrien wie beispielsweise Ellipsen, ebene Flächen oder ein Konus sind denkbar. Der radiale Abstand der zweiten Primärspulenwicklung von dem Zentrum der Gradientenspuleneinheit unterscheidet sich erfindungsgemäß von dem radialen Abstand der ersten Primärspulenwicklung. Die erste Primärspulenwicklung, die zweite Primärspulenwicklung und/oder eine Spulenwicklung im Allgemeinen sind typischerweise als Spulen ausgebildet, welche mit einer Spannungsquelle verbunden sind und/oder eine Induktivität aufweisen. Die erste Primärspule, die erste Sekundärspule und die erste Spannungsquelle sind typischerweise derart miteinander verschaltet, dass sie gemeinsam einen ersten Schaltkreis bilden. Die erste Primärspulenwicklung und die zweite Primärspulenwicklung weisen vorzugsweise einen ähnlich angeordneten und/oder verformten Leiter auf. Die erste Primärspulenwicklung und die zweite Primärspulenwicklung sind radial beabstandet.

Die zweite Primärspulenwicklung kann aus der ersten Primärspulenwicklung beispielsweise aus einer zentrischen Streckung hervorgehen. Die erste Primärspulenwicklung unterscheidet sich typischerweise von der zweiten Primärspulenwicklung. Die erste Primärspulenwicklung ist typischerweise ungleich der zweiten Primärspulenwicklung.

Der Vorteil der Gradientenspuleneinheit besteht darin, dass die Aufteilung der ersten Primärspule in eine erste Primärspulenwicklung und eine zweite Primärspulenwicklung bei unveränderter erster Spannungsquelle die Erzeugung einer höheren Gradientenamplitude, insbesondere einer höheren nominalen Gradientenamplitude, ermöglicht. Die Ansteuerung der erfindungsgemäßen Gradientenspuleneinheit kann mittels einer konventionellen ersten Gradientensteuereinheit und/oder einer konventionellen ersten Spanungsquelle ausgeführt werden. Insbesondere kann die erfindungsgemäße Gradientenspuleneinheit mit einer herkömmlichen Software angesteuert werden. Zudem ist für die Ansteuerung der erfindungsgemäßen Gradientenspuleneinheit mit zwei Primärspulenwicklungen nur eine Spannungsquelle und/oder ein Verstärker erforderlich. Der technische Aufwand ist demnach im Vergleich zu einer Gradientenspule umfassend mehrere mehrlagige Primärspulen und/oder Sekundärspulen gering.

Der ohmsche Widerstand der ersten Primärspule kann durch die Verwendung von der ersten Primärspulenwicklung und der zweiten Primärspulenwicklung im Vergleich zu einer herkömmlichen Primärspule mit nur einer Primärspulenwicklung reduziert sein, wodurch die erfindungsgemäße Gradientenspuleneinheit besonders effizient angesteuert werden kann. Ebenso kann der Magnetfeldgradient in die erste Richtung besonders effizient erzeugt werden.

Durch Vergrößerung der Gradientenamplitude, insbesondere der nominalen Gradientenamplitude, kann die erfindungsgemäße Gradientenspuleneinheit ein besonders großes Gradientenmoment pro Zeiteinheit bei unveränderter Slewrate erzeugen. Dadurch ist die Leistung der erfindungsgemäßen Gradientenspuleneinheit verbessert, ohne dass das Risiko einer Stimulation der peripheren Nerven des Patienten steigt. Die erste Primärspule erfordert mit der ersten Primärspulenwicklung und der zweiten Primärspulenwicklung im Vergleich zu einer Primärspule mit nur einer Primärspulenwicklung insbesondere nur einen geringen radialen räumlichen Mehrbedarf von etwa 5 mm bis 10 mm, sodass die erfindungsgemäße Gradientenspuleneinheit leicht in bestehende Magnetresonanzgeräte integriert werden kann. Im Allgemeinen erhöht sich die Effizienz einer Primärspule, und damit die Effizienz der Gradientenspuleneinheit, mit steigendem radialen Abstand zwischen der Primärspule und einer korrespondierenden Sekundärspule. Eine Gradientenspuleneinheit kann demnach effizienter angesteuert werden, je größer der radiale Abstand zwischen der Primärspule und der korrespondierenden Sekundärspule ist.

Eine Gradientenspuleneinheit weist typischerweise, auch wenn sie mehrere Primärspulen und Sekundärspulen umfasst, einen Spulenabstandsbereich zwischen der Menge der Primärspulen und der Menge der Sekundärspulen auf. Der Spulenabstandsbereich ist typischerweise frei von Spulenwicklungen. Die erfindungsgemäße Gradientenspuleneinheit kann demnach derart konstruiert sein, dass die erste und/oder die zweite Primärspulenwicklung innerhalb des bei einer herkömmlichen Gradientenspuleneinheit als Spulenabstandsbereich vorgesehenen Bereichs liegt. Die Effizienz der Gradientenspuleneinheit kann aufgrund der Aufteilung der ersten Primärspule dennoch beibehalten werden. Die räumlichen Abmessungen der erfindungsgemäßen Gradientenspuleneinheit können demnach so gewählt werden, dass sie den räumlichen Abmessungen einer konventionellen Gradientenspuleneinheit mit einer Primärspule mit nur einer Primärspulenwicklung entsprechen. Die erfindungsgemäße Gradientenspuleneinheit nutzt die räumlichen Abmessungen besonders effizient. Folglich kann auf eine Anpassung der Gradientensteuereinheit und/oder des Magnetresonanzgerätes an die erfindungsgemäße Gradientenspuleneinheit verzichtet werden. Die erfindungsgemäße Gradientenspuleneinheit kann demnach besonders kostengünstig eingesetzt werden.

Die Anordnung der ersten Primärspulenwicklung und der zweiten Primärspulenwicklung kann je nach Anforderung an eine geforderte maximale und/oder nominale Gradientenamplitude parallel oder in Reihe zur ersten Spannungsquelle sein. Die vorliegende Erfindung ist auf den Fall eingeschränkt, dass die Anordnung parallel ist. Für die erste Sekundärspule kann eine Reihenschaltung mit der ersten Primärspule gewählt werden, wodurch die erste Spannungsquelle beibehalten werden kann. Alternativ kann die erste Primärspule und/oder die erste Sekundärspule mit einer dritten Spannungsquelle verbunden sein, welche von der ersten Spannungsquelle verschieden ist. Der erste Schaltkreis kann demnach die erste Spannungsquelle und die dritte Spannungsquelle umfassen. Die erste Spannungsquelle und die dritte Spannungsquelle können in Serie oder parallel geschalten sein. Alternativ kann die erste Spannungsquelle mit der ersten Primärspule einen ersten Schaltkreis bilden und die dritte Spannungsquelle mit der ersten Sekundärspule einen dritten Schaltkreis bilden. Durch die Verwendung der ersten Spannungsquelle und der dritten Spannungsquelle kann besonders effizient die Slewrate auf beispielsweise über 200 T/m/s erhöht werden. Dies ist besonders vorteilhaft für Verfahren zur MR-Bildgebung, welche eine geringe maximale Gradientenamplitude von beispielsweise weniger als 15 mT/m mit einer minimalen Anstiegszeit erfordern, wie beispielsweise Spiralsequenzen oder blipped EPIs. Die höhere Verlustleistung aufgrund der höheren Frequenzen der Gradientenpulse kann durch die Verdopplung der Kühlleistung ausgeglichen werden.

Eine Ausführungsform der Gradientenspuleneinheit und/oder des Gradientensystems sieht vor, dass die Gradientenspuleneinheit eine der ersten Primärspulenwicklung zugeordnete erste Kühlungslage und eine der zweiten Primärspulenwicklung zugeordnete zweite Kühlungslage umfasst.

Eine Kühlungslage ist typischerweise eine Anordnung, welche ein Kühlmedium umfasst. Das Kühlmedium kann vorzugsweise innerhalb der Kühlungslage zirkulieren. Die Zirkulation des Kühlmediums kann aktiv und/oder passiv erfolgen. Die Kühlungslage kann hierfür Kühlschläuche und/oder weitere Hohlformen umfassen, innerhalb welchen Kühlschläuchen und/oder weiteren Hohlformen das Kühlmedium zirkulieren kann. Das Kühlmedium ist vorzugsweise eine Flüssigkeit oder ein Gas. Das Kühlmedium weist vorzugsweise eine besonders hohe Wärmekapazität auf. Ist eine Kühlungslage, beispielsweise die erste Kühlungslage, einer Spulenwicklung, beispielsweise der ersten Primärspulenwicklung zugeordnet, so ist die Kühlungslage dazu ausgebildet, im Betrieb der Gradientenspuleneinheit, insbesondere bei Ansteuerung der Spulenwicklung mittels eines Stromes, beispielsweise bei Induktion des ersten Stromes, entstehende Wärme abzuleiten. Die einer Spulenwicklung zugeordnete Kühlungslage liegt vorzugsweise großflächig an der Spulenwicklung an und/oder ist mit dieser großflächig benachbart und/oder verwoben. Die Kühlkapazität einer Kühlungslage wird vorrangig, insbesondere zu zumindest 70%, bevorzugt zu zumindest 80%, für die Kühlung der ihr zugeordneten Spulenwicklung, insbesondere bei deren Betrieb, verwendet.

Die erste Kühlungslage ist typischerweise von der zweiten Kühlungslage verschieden. Die Kühlkapazität der ersten Kühlungslage entspricht typischerweise näherungsweise der Kühlkapazität der zweiten Kühlungslage. Die erste Kühlungslage ist vorzugsweise analog zur zweiten Kühlungslage ausgestaltet. Insbesondere können die erste Kühlungslage und die zweite Kühlungslage eine gleiche Funktionalität und/oder Geometrie aufweisen. Die Zuordnung und/oder Verbindung und/oder Verflechtung der zweiten Kühlungslage mit der zweiten Primärspulenwicklung ist vorzugsweise analog zur Zuordnung und/oder Verbindung und/oder Verflechtung der ersten Kühlungslage mit der ersten Primärspulenwicklung ausgestaltet. Gemäß dieser Ausführungsform sind der ersten Primärspule demnach zwei Kühlungslagen zugeordnet, sodass die erste Primärspule besonders effizient gekühlt werden kann. Hierdurch kann die Kühlkapazität im Vergleich zu einer herkömmlichen Gradientenspuleneinheit mit einer herkömmlichen, nicht in eine erste Primärspulenwicklung und eine zweite Primärspulenwicklung unterteilte, Primärspule, verdoppelt werden. Hierdurch kann die nominale Gradientenamplitude um etwa 40% erhöht werden. Dadurch kann die Gradientenspuleneinheit besonders effizient angesteuert werden und der Magnetfeldgradient in die erste Richtung besonders effizient erzeugt werden.

Eine Ausführungsform des Gradientensystems sieht vor, dass die Gradientenspuleneinheit eine zweite Primärspule mit einer dritten Primärspulenwicklung umfasst, welche dritte Primärspulenwicklung mit einer zweiten Spannungsquelle in Serie geschaltet ist und dazu ausgebildet ist, einen Magnetfeldgradient in eine zweite Richtung zu erzeugen, sofern die zweite Spannungsquelle einen zweiten Strom induziert.

Die zweite Richtung ist vorzugsweise orthogonal zur ersten Richtung. Die zweite Primärspule entspricht gemäß dieser Ausführungsform einer typischerweise herkömmlichen Gradientenspuleneinheit. Gemäß dieser Ausführungsform kombiniert die Gradientenspuleneinheit eine herkömmliche Primärspule mit der ersten Primärspule umfassend die erste Primärspulenwicklung und die zweite Primärspulenwicklung. Die erste Primärspule unterscheidet sich von der zweiten Primärspule. Die zweite Primärspule umfasst zusätzlich zur dritten Primärspulenwicklung vorzugsweise keine weitere Primärspulenwicklung. Die dritte Primärspulenwicklung ist vorzugsweise die einzige von der zweiten Primärspule umfasste Primärspulenwicklung.

In die erste Richtung kann demnach ein Magnetfeldgradient mit einer besonders großen Amplitude erzeugt werden. Dies ist besonders für eine funktionelle MR-Bildgebung, beispielsweise mittels der BOLD-Methode, vorteilhaft, da der Kopf jedes Patienten innerhalb der Gradientenspuleneinheit typischerweise gleich orientiert und/oder positioniert ist. Demnach kann eine auszuspielende MR-Steuerungssequenz derart gewählt sein, dass die Richtung mit größter Anforderung an die Gradientenspuleneinheit der ersten Richtung entspricht. In der echoplanaren MR-Bildgebung ist dies beispielsweise die x-Richtung, also typischerweise die horizontale Richtung senkrecht zur Zylinderachse der Gradientenspuleneinheit. Aus physiologischen Gründen hat sich eine konstante Orientierung des Auslesegradienten in die x-Richtung etabliert. Optimale Ergebnisse werden erzielt, sofern die Amplitude und/oder die Slewrate des Magnetfeldgradienten in die x-Richtung besonders hoch sind. Die erste Richtung entspricht vorzugsweise der x-Richtung. Die erste Richtung kann auch einer anderen Richtung entsprechen, in welche Richtung eine MR-Steuerungssequenz besonders hohe Anforderungen an die Gradientenspuleneinheit stellt.

Der vereinfachte Aufbau der zweiten Primärspule ermöglicht eine selektive Verbesserung der ersten Primärspule. Insbesondere kann die zweite Primärspule platzsparend und/oder kostengünstig gefertigt werden. Die Effizienz der zweiten Primärspule entspricht vorzugsweise der Effizienz einer herkömmlichen Primärspule. Die Gradientenspuleneinheit kann zumindest eine weitere Primärspule mit einer dieser weiteren zugeordneten Sekundärspule umfassen, welche weitere Primärspule analog zur zweiten Primärspule aufgebaut ist. Eine Gradientenspuleneinheit gemäß dieser Ausführungsform fokussiert auf eine Verbesserung und/oder Erhöhung der Effizienz der ersten Primärspule, welche einen Magnetfeldgradienten in die erste Richtung erzeugt. Vorzugweise erfordern mehrere MR-Steuerungssequenzen eine besonders hohe Gradientenamplitude in die erste Richtung. Der Spulenabstandsbereich wird aufgrund einer zusätzlichen Primärspulenwicklung der zweiten Primärspule nicht verkleinert. Dadurch wird die Effizienz der Gradientenspuleneinheit zumindest beibehalten und/oder vergrößert.

Eine Ausführungsform des Gradientensystems sieht vor, dass bei Vorhandensein des ersten Stromes eine Stromdichte in der ersten Primärspulenwicklung und/oder der zweiten Primärspulenwicklung maximal 70% einer Stromdichte in der dritten Primärspulenwicklung bei Vorhandensein des zweiten Stromes beträgt.

Dabei sind erfindungsgemäss die erste Primärspulenwicklung und die zweite Primärspulenwicklung parallel zu der ersten Spannungsquelle geschalten. Der erste Strom geht von der ersten Spannungsquelle aus und teilt sich typischerweise auf die erste Primärspulenwicklung und die zweite Primärspulenwicklung auf. Basierend auf dem ersten Strom wird von der ersten Primärspulenwicklung und der zweiten Primärspulenwicklung der erste Magnetfeldgradient erzeugt. Erzeugt die dritte Primärspulenwicklung basierend auf dem zweiten Strom den zweiten Magnetfeldgradient und entspricht die Amplitude des zweiten Magnetfeldgradienten der Amplitude des ersten Magnetfeldgradienten, so beträgt vorzugsweise die Stromdichte in der ersten Primärspulenwicklung und/oder der zweiten Primärspulenwicklung maximal 70%, bevorzugt maximal 60%, besonders bevorzugt maximal 55% der Stromdichte in der dritten Primärspulenwicklung. Entspricht der erste Strom dem zweiten Strom, so beträgt vorzugsweise die Stromdichte in der ersten Primärspulenwicklung und/oder der zweiten Primärspulenwicklung maximal 70%, bevorzugt maximal 60%, besonders bevorzugt maximal 55% der Stromdichte in der dritten Primärspulenwicklung.

Gemäß dieser Ausführungsform ist der ohmsche Widerstand der ersten Primärspule maximal 70%, bevorzugt maximal 60%, besonders bevorzugt maximal 55% so groß wie der ohmsche Widerstand der zweiten Primärspule. Dadurch kann bei gleicher Leistungszufuhr die erste Primärspule einen Magnetfeldgradienten mit höherer Amplitude erzeugen als die zweite Primärspule.

Eine Ausführungsform der Gradientenspuleneinheit und/oder des Gradientensystems sieht vor, dass die Gradientenspuleneinheit eine der dritten Primärspulenwicklung zugeordnete dritte Kühlungslage umfasst. Der zweiten Primärspule ist demnach vorzugsweise genau eine Kühlungslage zugeordnet. Die Kühlkapazität der dritten Kühlungslage entspricht typischerweise näherungsweise der Kühlkapazität der zweiten und/oder ersten Kühlungslage. Die dritte Kühlungslage ist vorzugsweise analog zur ersten Kühlungslage ausgestaltet. Die Zuordnung und/oder Verbindung und/oder Verflechtung der dritten Kühlungslage mit der dritten Primärspulenwicklung ist vorzugsweise analog zur Zuordnung und/oder Verbindung und/oder Verflechtung der ersten Kühlungslage mit der ersten Primärspulenwicklung ausgestaltet.

Der vereinfachte Aufbau der zweiten Primärspule und die Zuordnung einer dritten Kühlungslage ermöglicht eine selektive Verbesserung der ersten Primärspule. Insbesondere kann die zweite Primärspule mit der dritten Kühlungslage platzsparend und/oder kostengünstig gefertigt werden. Die Effizienz der zweiten Primärspule entspricht vorzugsweise der Effizienz einer herkömmlichen Primärspule.

Eine Ausführungsform des Gradientensystems sieht vor, dass die erste Sekundärspule eine erste Sekundärspulenwicklung umfasst, welche erste Sekundärspulenwicklung mit der ersten Spannungsquelle in Serie geschaltet ist und eine Abschirmung des Magnetfeldgradienten in die erste Richtung bewirkt, sofern die erste Spannungsquelle den ersten Strom induziert.

Die erste Sekundärspule bildet typischerweise gemeinsam mit der ersten Primärspule und der ersten Spannungsquelle einen ersten Schaltkreis, wobei dieser vorzugsweise einer Serienschaltung entspricht. Die erste Sekundärspule entspricht vorzugsweise einer konventionellen Sekundärspule, welche nur eine Sekundärspulenwicklung umfasst. Der ersten Sekundärspule ist vorzugsweise eine fünfte Kühlungslage zugeordnet. Im Allgemeinen weist eine Sekundärspule typischerweise eine halbe Windungszahl der ihr zugeordneten Primärspule auf. Die Windungszahl der ersten Sekundärspule, insbesondere der ersten Sekundärspulenwicklung, ist demnach vorzugsweise halb so groß wie die Windungszahl der ersten Primärspule. Die Amplitude des mittels der ersten Sekundärspule zu erzeugenden Magnetfeldes ist typischerweise geringer als die Amplitude des mittels der ersten Primärspule zu erzeugenden Magnetfeldgradienten. Die Leistungszufuhr und/oder Leistungsabgabe an die erste Sekundärspule ist demnach typischerweise geringer als an die erste Primärspule. Folglich kann bei der ersten Sekundärspule auf eine Auslegung analog zur ersten Primärspule umfassend zwei Spulenwicklungen und zwei Kühlungslagen verzichtet werden, ohne dass die Effizienz der Gradientenspuleneinheit limitiert wird. Gemäß dieser Ausführungsform kann die Gradientenspuleneinheit besonders platzsparend ausgestaltet sein und kostengünstig gefertigt werden.

Die Erfindung sieht vor, dass die erste Primärspulenwicklung und die zweite Primärspulenwicklung parallel zu der ersten Spannungsquelle geschalten sind. Die Parallelschaltung bewirkt eine Reduktion der Stromdichte in der ersten Primärspulenwicklung und/oder der zweiten Primärspulenwicklung. Der erste Strom, welcher zu einer Erzeugung des Magnetfeldgradienten in die erste Richtung benötigt wird, entspricht vorzugsweise dem von einer herkömmlichen ersten Primärspule benötigten Strom zur Erzeugung des Magnetfeldgradienten in die erste Richtung. Der ohmsche Widerstand der ersten Primärspule wird reduziert bei gleichzeitig verbesserter Kühlkapazität. Folglich ist die Gradientenspuleneinheit besonders effizient.

Eine sich ausserhalb des durch die Ansprüche definierten Schutzumfanges befindliche Alternative des Gradientensystems sieht vor, dass die erste Primärspulenwicklung und die zweite Primärspulenwicklung in Serie mit der ersten Spannungsquelle geschalten sind. Die erste Primärspulenwicklung und die zweite Primärspulenwicklung weisen dabei vorzugsweise gemeinsam eine Windungszahl auf, welche näherungsweise der Windungszahl einer von einer herkömmlichen Gradientenspuleneinheit umfassten Primärspule entspricht. Die erste Primärspulenwicklung und die zweite Primärspulenwicklung weisen dabei vorzugsweise jeweils eine Windungszahl auf, welche näherungsweise der halben Windungszahl einer von einer herkömmlichen Gradientenspuleneinheit umfassten Primärspule entspricht. Die Aufteilung der ersten Primärspule in die erste Primärspulenwicklung und die zweite Primärspulenwicklung ermöglicht eine erhöhte Kühlkapazität für von der Primärspule umfasste Leiter, wodurch die Effizienz der Gradientenspuleneinheit verbessert wird.

Die erste Spannungsquelle ist mit der von der Gradientenspuleneinheit umfassten ersten Primärspulenwicklung und zweiten Primärspulenwicklung verschaltet. Die erste Primärspulenwicklung und die zweite Primärspulenwicklung sind gemeinsam dazu ausgebildet, einen Magnetfeldgradient in eine erste Richtung zu erzeugen, sofern die erste Spannungsquelle einen ersten Strom induziert.

Das Gradientensystem kann eine zweite Spannungsquelle umfassen, welche mit der von der Gradientenspuleneinheit umfassten dritten Primärspulenwicklung in Serie geschaltet ist, wobei die dritte Primärspulenwicklung dazu ausgebildet ist, einen Magnetfeldgradient in eine zweite Richtung zu erzeugen, sofern die zweite Spannungsquelle einen zweiten Strom induziert. Die erste Spannungsquelle ist vorzugsweise speziell für die Anforderungen der erfindungsgemäßen ersten Primärspule ausgelegt. Die zweite Spannungsquelle entspricht vorzugsweise einer Spannungsquelle für einen Betrieb einer herkömmlichen Gradientenspuleneinheit.

Des Weiteren geht die Erfindung aus von einem Magnetresonanzgerät umfassend ein erfindungsgemäßes Gradientensystem, einen Hauptmagneten und eine Hochfrequenzantenneneinheit. Das erfindungsgemäße Magnetresonanzgerät umfasst demnach zumindest

- einen Hauptmagneten,
- eine Hochfrequenzantenneneinheit,
- eine erste Primärspule mit einer ersten Primärspulenwicklung und mit einer zweiten Primärspulenwicklung, welche jeweils auf einer Mantelfläche eines Kreiszylinders angeordnet und zueinander radial beabstandet sind,
- eine erste Sekundärspule, und
- eine mit der ersten Primärspulenwicklung und der zweiten Primärspulenwicklung verschaltete erste

Spannungsquelle, wobei die erste Primärspulenwicklung und die zweite Primärspulenwicklung parallel zu der ersten Spannungsquelle geschaltet sind.

Die erste Primärspulenwicklung und die zweite Primärspulenwicklung sind gemeinsam dazu ausgebildet einen Magnetfeldgradient in eine erste Richtung zu erzeugen, sofern die erste Spannungsquelle einen ersten Strom induziert.

Die Gradientenspuleneinheit des erfindungsgemässen Gradientensystems kann auch separat von dem Magnetresonanzgerät installiert sein. Das erfindungsgemäße Gradientensystem kann mit dem Magnetresonanzgerät verbunden sein. Ausführungsformen des erfindungsgemäßen Magnetresonanzgerätes sind analog zu den Ausführungsformen des erfindungsgemäßen Gradientensystems ausgebildet. Das Magnetresonanzgerät kann weitere Steuerungskomponenten aufweisen, welche nötig und/oder vorteilhaft sind. Auch kann das Magnetresonanzgerät dazu ausgebildet sein, Steuerungssignale zu senden und/oder Steuerungssignale zu empfangen und/oder zu verarbeiten.

Eine Ausführungsform des Magnetresonanzgeräts sieht vor, dass ein definierter hohlzylindrischer Abstandsbereich zwischen dem Hauptmagnet und der Hochfrequenzantenneneinheit die Gradientenspuleneinheit mit der ersten Primärspule umfassend die erste Primärspulenwicklung und die zweite Primärspulenwicklung umfasst. Für eine Integration der Gradientenspuleneinheit in das Magnetresonanzgerät ist demnach erforderlich, dass die äußere Abmessung der Gradientenspuleneinheit maximal dem hohlzylindrischen Abstandsbereich entspricht. In diesem Fall kann die Gradientenspuleneinheit eine für das Magnetresonanzgerät vorgesehene herkömmliche Gradientenspuleneinheit ersetzen und/oder mit dieser getauscht werden. Folglich kann dann auch ein schon bestehendes Magnetresonanzgerät mit einer Gradientenspuleneinheit des erfindungsgemässen Gradientensystems nachgerüstet werden. Zudem ist keine Anpassung eines zur Gradientenspuleneinheit zusätzlichen Bauteils des Magnetresonanzgerätes erforderlich.

Des Weiteren geht die Erfindung aus von einem Verfahren zu einer Auslegung einer ersten Primärspulenwicklung und einer zweiten Primärspulenwicklung einer ersten Primärspule einer Gradientenspuleneinheit eines Gradientensystems, welches eine erste Spannungsquelle umfasst, wobei die erste Primärspulenwicklung und die zweite Primärspulenwicklung, jeweils angeordnet auf einer Mantelfläche eines Kreiszylinders und zueinander radial beabstandet, parallel mit der ersten Spannungsquelle verschaltet sind, gemäß den folgenden Verfahrensschritten:

- Vorgabe eines Magnetfeldgradienten in eine erste Richtung
- Bestimmung einer Geometrie und/oder einer elektrischen Eigenschaft der ersten Primärspulenwicklung und der zweiten Primärspulenwicklung derart, dass bei Induktion eines ersten Stromes durch die erste Spannungsquelle gemeinsam von der ersten Primärspulenwicklung und der zweiten Primärspulenwicklung der Magnetfeldgradient in die erste Richtung erzeugt wird.

Das erfindungsgemäße Verfahren beschreibt demnach eine Methode zum Design einer erfindungsgemäßen Gradientenspuleneinheit. Ausgehend von einem Magnetfeldgradienten in eine erste Richtung wird die Geometrie und/oder elektrische Eigenschaften der ersten Primärspule, typischerweise mittels einer Optimierungsmethode, bestimmt. Vorzugsweise werden dabei auch die Geometrie und/oder elektrische Eigenschaften der ersten Sekundärspule bestimmt. Dabei wird typischerweise zumindest eine Randbedingung berücksichtigt. Eine Randbedingung kann beispielsweise eine Linearität und/oder Sensitivität des Magnetfeldgradienten in die erste Richtung und/oder eine maximale Stromdichte umfassen. Der Magnetfeldgradienten in die erste Richtung entspricht typischerweise einem von einer herkömmlichen Gradientenspule erzeugten Magnetfeldgradienten in die erste Richtung.

Die Vorteile des erfindungsgemäßen Magnetresonanzgeräts und des erfindungsgemäßen Verfahrens entsprechen im Wesentlichen den Vorteilen des erfindungsgemäßen Gradientensystems, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt. Weitere Vorteile, Merkmale und Einzelheiten der Erfindung ergeben sich aus den im Folgenden beschriebenen Ausführungsbeispielen sowie anhand der Zeichnungen.

Es zeigen:

Fig. 1      eine schematische Darstellung eines erfindungsgemäßen Magnetresonanzgerätes,

Fig. 2      eine schematische Darstellung einer aus dem Stand der Technik bekannten Gradientenspuleneinheit,

Fig. 3      eine schematische Darstellung einer ersten Gradientenspuleneinheit,

Fig. 4      ein Schaltbild einer ersten Ausführungsform eines erfindungsgemäßen Gradientensystems,

Fig. 5      ein Schaltbild eines nicht-erfindungsgemässen Gradientensystems, und

Fig. 6      ein Ablaufdiagramm eines erfindungsgemäßen Verfahrens.

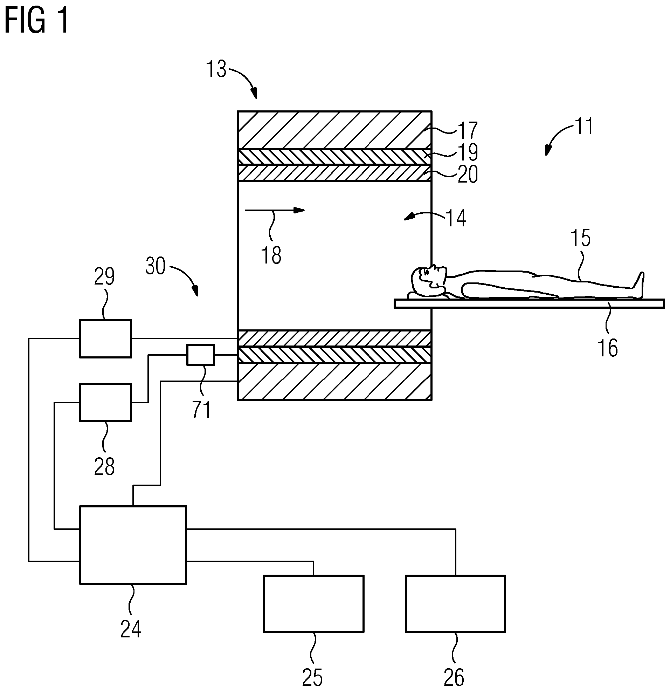

Figur 1 zeigt ein erfindungsgemäßes Magnetresonanzgerät 11. Das Magnetresonanzgerät 11 umfasst eine von einer Magneteinheit 13 gebildeten Detektoreinheit mit einem Hauptmagneten 17 zu einem Erzeugen eines starken und insbesondere konstanten Hauptmagnetfelds 18. Zudem weist das Magnetresonanzgerät 11 einen zylinderförmigen Patientenaufnahmebereich 14 zu einer Aufnahme eines Patienten 15 auf, wobei der Patientenaufnahmebereich 14 in einer Umfangsrichtung von der Magneteinheit 13 zylinderförmig umschlossen ist. Der Patient 15 kann mittels einer Patientenlagerungsvorrichtung 16 des Magnetresonanzgeräts 11 in den Patientenaufnahmebereich 14 geschoben werden. Die Patientenlagerungsvorrichtung 16 weist hierzu einen Patententisch auf, der bewegbar innerhalb des Magnetresonanzgeräts 11 angeordnet ist.

Die Magneteinheit 13 weist weiterhin eine Hochfrequenzantenneneinheit 20, welche im gezeigten Fall als fest in das Magnetresonanzgerät 11 integrierte Körperspule ausgebildet ist, und eine Hochfrequenzantennensteuereinheit 29 zu einer Anregung einer Polarisation, die sich in dem von dem Hauptmagneten 17 erzeugten Hauptmagnetfeld 18 einstellt, auf. Die Hochfrequenzantenneneinheit 20 wird von der Hochfrequenzantennensteuereinheit 29 angesteuert und strahlt hochfrequente Hochfrequenz-Pulse in einen Untersuchungsraum, der im Wesentlichen von dem Patientenaufnahmebereich 14 gebildet ist, ein. Zwischen dem Hauptmagnet 17 und der Hochfrequenzantenneneinheit 20 weist das Magnetresonanzgerät 11 einen hohlzylindrischen Abstandsbereich auf. Der hohlzylindrische Abstandsbereich ist typischerweise derart ausgestaltet, dass darin eine herkömmliche, also nicht erfindungsgemäße, Gradientenspuleneinheit für eine Ortskodierung positioniert werden kann. Die im erfindungsgemässen Gradientensystem enthaltene Gradientenspuleneinheit 19 hat eine derartige äußere Geometrie und/oder äußere Abmessung, dass diese im hohlzylindrischen Abstandsbereich zwischen dem Hauptmagnet 17 und der Hochfrequenzantenneneinheit 20 angeordnet werden kann. Die Magneteinheit 13 weist eine derartige Gradientenspuleneinheit 19 auf. Die Gradientenspuleneinheit 19 ist mit der ersten Spannungsquelle 71 verbunden und wird mittels einer Gradientensteuereinheit 28 angesteuert. Die Gradientenspuleneinheit 19 und die erste Spannungsquelle 71 bilden zusammen das erfindungsgemäße Gradientensystem 30. Das Gradientensystem 30 kann auch die Gradientensteuereinheit 28 umfassen.

Zu einer Steuerung des Hauptmagneten 17, der Gradientensteuereinheit 28 und der Hochfrequenzantennensteuereinheit 29 weist das Magnetresonanzgerät 11 eine Steuerungseinheit 24 auf. Die Steuerungseinheit 24 steuert zentral das Magnetresonanzgerät 11, wie beispielsweise das Durchführen von MR-Steuerungssequenzen. Das Magnetresonanzgerät 11 weist eine Anzeigeeinheit 25 auf. Zudem weist das Magnetresonanzgerät 11 eine Eingabeeinheit 26 auf, mittels derer Informationen und/oder Steuerungsparameter während eines Messvorgangs von einem Benutzer eingegeben werden können. Die Steuerungseinheit 24 kann die Gradientensteuereinheit 28 und/oder Hochfrequenzantennensteuereinheit 29 und/oder die Anzeigeeinheit 25 und/oder die Eingabeeinheit 26 umfassen.

Das dargestellte Magnetresonanzgerät 11 kann selbstverständlich weitere Komponenten umfassen, die Magnetresonanzgeräte 11 gewöhnlich aufweisen. Eine allgemeine Funktionsweise eines Magnetresonanzgeräts 11 ist zudem dem Fachmann bekannt, so dass auf eine detaillierte Beschreibung der weiteren Komponenten verzichtet wird.

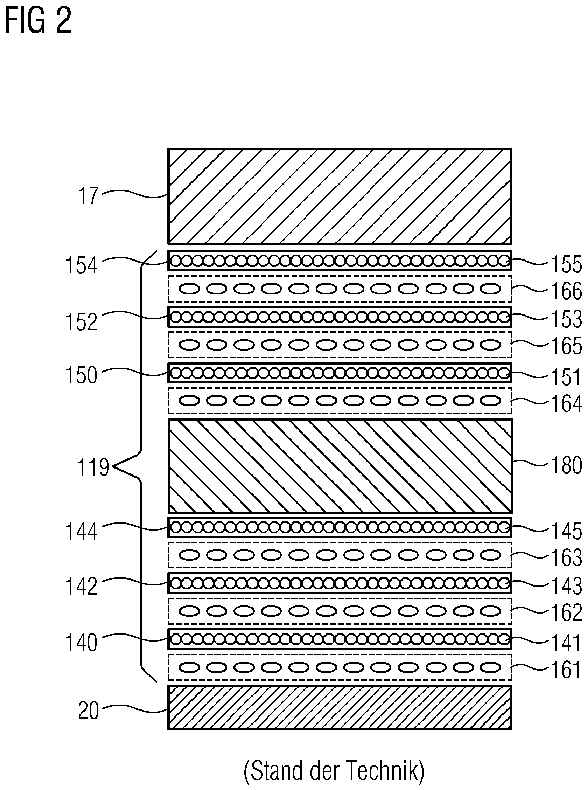

Figur 2 zeigt einen radialen Aufbau einer aus dem Stand der Technik bekannten, also herkömmlichen Gradientenspuleneinheit 119 umgeben von dem Hauptmagnet 17 und der Hochfrequenzantenneneinheit 20 eines Magnetresonanzgerätes 11. Die Ansicht der herkömmlichen Gradientenspuleneinheit 119 ist im Vergleich zur Ansicht in Figur 1 auf eine durch die Zylinderachse der herkömmlichen Gradientenspuleneinheit 119 begrenzte Halbebene reduziert. Zwischen dem Hauptmagnet 17 und der Hochfrequenzantenneneinheit 11 ist ein definierter hohlzylindrischer Abstandsbereich, welcher von der herkömmlichen Gradientenspuleneinheit 119 ausgefüllt wird.

Die Komponenten der herkömmlichen Gradientenspuleneinheit 119 sind radial nach außen gemäß der folgenden Reihenfolge angeordnet:

- eine erste Kühlungslage 161 für eine erste Primärspule 140,

- die erste Primärspule 140 mit einer ersten Primärspulenwicklung 141,
- eine zweite Kühlungslage 162 für eine zweite Primärspule 142,
- die zweite Primärspule 142 mit einer zweiten Primärspulenwicklung 143,
- eine dritte Kühlungslage 163 für eine dritte Primärspule 144,
- die dritte Primärspule 144 mit einer dritten Primärspulenwicklung 145,
- ein Spulenabstandsbereich 180, welcher die Primärspulen 140, 142, 144 von den Sekundärspulen 150, 152, 154 trennt,
- eine vierte Kühlungslage 164 für eine erste Sekundärspule 150,
- die erste Sekundärspule 150 mit einer ersten Sekundärspulenwicklung 151,
- eine zweite Kühlungslage 165 für eine zweite Sekundärspule 152,
- die zweite Sekundärspule 152 mit einer zweiten Sekundärspulenwicklung 153,
- eine dritte Kühlungslage 166 für eine dritte Sekundärspule 154,
- die dritte Sekundärspule 154 mit einer dritten Sekundärspulenwicklung 155.

Die erste Primärspule 140 ist dazu ausgebildet, einen Magnetfeldgradienten in eine erste Raumrichtung zu erzeugen und die erste Sekundärspule 150 ist dazu ausgebildet, den Magnetfeldgradienten in die erste Raumrichtung abzuschirmen. Die zweite Primärspule 142 ist dazu ausgebildet, einen Magnetfeldgradienten in eine zweite Raumrichtung zu erzeugen und die zweite Sekundärspule 152 ist dazu ausgebildet, den Magnetfeldgradienten in die zweite Raumrichtung abzuschirmen. Die dritte Primärspule 144 ist dazu ausgebildet, einen Magnetfeldgradienten in eine dritte Raumrichtung zu erzeugen und die dritte Sekundärspule 154 ist dazu ausgebildet, den Magnetfeldgradienten in die dritte Raumrichtung abzuschirmen.

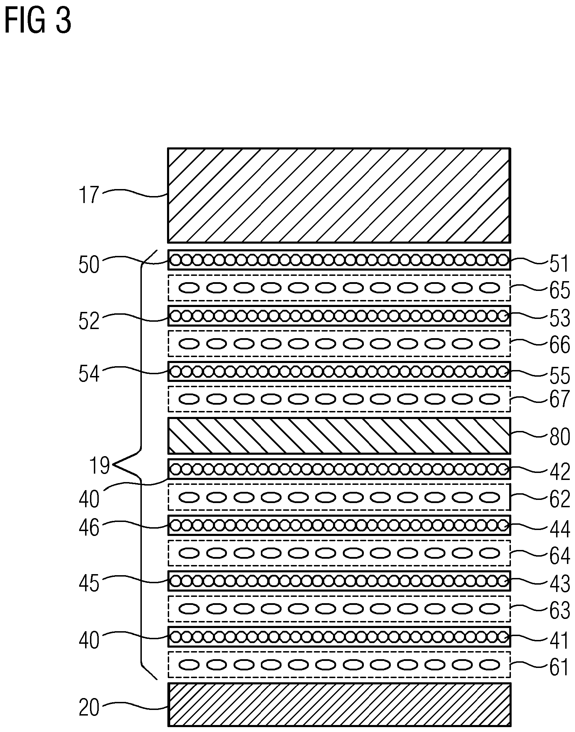

Figur 3 zeigt einen radialen Aufbau einer im erfindungsgemässen Gradientensystem verwendbaren Gradientenspuleneinheit 19 umgeben von dem Hauptmagnet 17 und der Hochfrequenzantenneneinheit 20 des Magnetresonanzgerätes 11. Die Ansicht der Gradientenspuleneinheit 19 ist im Vergleich zur Ansicht in Figur 1 auf eine durch die Zylinderachse der Gradientenspuleneinheit 19 begrenzte Halbebene reduziert. Zwischen dem Hauptmagnet 17 und der Hochfrequenzantenneneinheit 11 ist ein definierter hohlzylindrischer Abstandsbereich, welcher vorzugsweise dem definierten hohlzylindrischen Abstandsbereich der herkömmlichen Ausführung in Figur 2 entspricht. Dieser definierte hohlzylindrische Abstandsbereich wird vorzugsweise von der Gradientenspuleneinheit 19 ausgefüllt. Die äußeren geometrischen Abmessungen der

Gradientenspuleneinheit 19 entsprechen vorzugsweise den äußeren geometrischen Abmessungen der herkömmlichen Gradientenspuleneinheit 119.

Die Gradientenspuleneinheit 19 weist die folgenden, radial nach außen gemäß der folgenden Reihenfolge angeordneten Komponenten auf:

- eine erste Kühlungslage 61 für eine von der ersten Primärspule 40 umfasste ersten Primärspulenwicklung 41,
- die erste Primärspulenwicklung 41,
- eine dritte Kühlungslage 63 für eine zweite Primärspule 45,
- die zweite Primärspule 45 mit einer dritten Primärspulenwicklung 43,
- eine vierte Kühlungslage 64 für eine dritte Primärspule 46,
- die dritte Primärspule 46 mit einer vierten Primärspulenwicklung 44,
- eine zweite Kühlungslage 62 für eine von der ersten Primärspule 40 umfasste zweite Primärspulenwicklung 42,
- die zweite Primärspulenwicklung 42,
- ein Spulenabstandsbereich 80, welcher die Primärspulen 40, 45, 46 von den Sekundärspulen 50, 52, 54 trennt,
- eine siebte Kühlungslage 67 für eine zweite Sekundärspule 54,
- die zweite Sekundärspule 54 mit einer zweiten Sekundärspulenwicklung 55,
- eine sechste Kühlungslage 66 für eine dritte Sekundärspule 52,
- die dritte Sekundärspule 52 mit einer dritten Sekundärspulenwicklung 53,
- eine fünfte Kühlungslage 65 für eine erste Sekundärspule 50,
- die erste Sekundärspule 50 mit einer ersten Sekundärspulenwicklung 51.

Eine Kühlungslage für eine Spule und/oder für eine Spulenwicklung ist dieser Spule und/oder Spulenwicklung zugeordnet. Beispielsweise ist demnach die erste Kühlungslage 61 der ersten Primärspulenwicklung 41 zugeordnet. Die von der Gradientenspuleneinheit 19 umfassten Kühlungslagen 61, 62, 63, 64, 65, 66, 67 sind demnach jeweils einer Spule oder Spulenwicklung zugeordnet, sind demnach dazu ausgebildet, vorrangig die ihnen zugeordnete Spule oder Spulenwicklung zu kühlen. Hierfür kann die Spule oder Spulenwicklung mit ihrer zugeordneten Kühlungslage verwoben angeordnet sein und/oder in der oben aufgeführten Reihenfolge vertauscht sein.

Die erste Primärspule 40 ist dazu ausgebildet, einen Magnetfeldgradienten in eine erste Raumrichtung zu erzeugen und die erste Sekundärspule 50 ist dazu ausgebildet, den Magnetfeldgradienten in die erste Raumrichtung abzuschirmen. Die zweite Primärspule 45 ist dazu ausgebildet, einen Magnetfeldgradienten in eine zweite Raumrichtung zu erzeugen und die zweite Sekundärspule 54 ist dazu ausgebildet, den Magnetfeldgradienten in die zweite Raumrichtung abzuschirmen. Die dritte Primärspule 46 ist dazu ausgebildet, einen Magnetfeldgradienten in eine dritte Raumrichtung zu erzeugen und die dritte Sekundärspule 52 ist dazu ausgebildet, den Magnetfeldgradienten in die dritte Raumrichtung abzuschirmen.

Der von der ersten Sekundärspule 50 erzeugte Magnetfeldgradient weist typischerweise die gleiche Amplitude mit verschiedener Polarität des von der ersten Primärspule 40 erzeugten Magnetfeldgradienten auf, sodass sich die beiden Magnetfeldgradienten aufheben würden, wenn die erste Primärspule 40 und die erste Sekundärspule 50 an gleicher Position angeordnet wären. Aufgrund eines radialen Abstandes zwischen der ersten Primärspule 40 und der ersten Sekundärspule 50 wird im Patientenaufnahmebereich 14 bei Ansteuerung der ersten Primärspule 40 und der ersten Sekundärspule 50 durch Induktion des ersten Stromes ein Magnetfeldgradient erzeugt, welcher ungleich null ist. Je größer der radiale Abstand zwischen der ersten Primärspule 40 und der ersten Sekundärspule 50 ist, desto größer ist die Effizienz der Gradientenspuleneinheit 19. Die Effizienz einer Gradientenspuleneinheit 19 kann durch den Quotient der Stärke des Magnetfeldgradienten im Patientenaufnahmebereich 14 und der Stärke des ersten Stromes angegeben werden. Der in diesem Absatz aufgeführte Zusammenhang gilt allgemein für eine Primärspule und einer der Primärspule zugeordneten Sekundärspule.

Gemäß dem in Figur 3 dargestellten Aufbau bleibt im Vergleich zur herkömmlichen Gradientenspule 119 der radiale Abstand zwischen der zweiten Primärspule 45 und der zweiten Sekundärspule 54, sowie der radiale Abstand zwischen der dritten Primärspule 46 und der dritten Sekundärspule 52 erhalten, demnach vorzugsweise gleich groß. Der radiale Abstand zwischen der mittleren radialen Position der ersten Primärspule 40 und der ersten Sekundärspule 50 vergrößert sich. Der Spulenabstandsbereich 80 weist eine geringere radiale Ausdehnung auf als der Spulenabstandsbereich 180 der herkömmlichen Gradientenspuleneinheit 119. Vorzugsweise entsprechen die äußeren Abmessungen des Spulenabstandsbereichs 80, der zweiten Kühlungslage 62 und der zweite Primärspulenwicklung 42 zusammen den äußeren Abmessungen des Spulenabstandsbereich 180 der herkömmlichen Gradientenspuleneinheit 119.

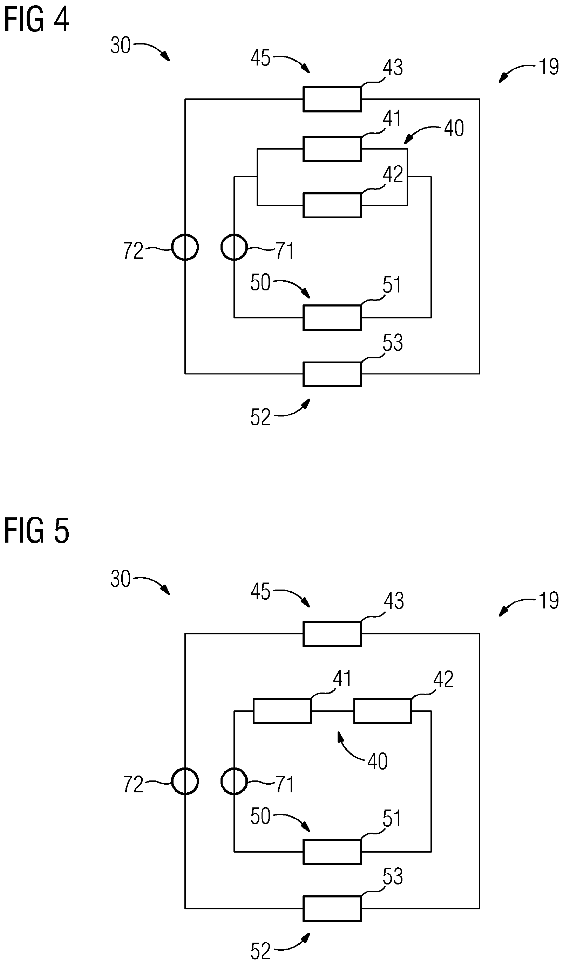

Figur 4 zeigt ein Schaltbild einer ersten Ausführungsform eines erfindungsgemäßen Gradientensystems 30. Das Gradientensystem weist eine erste Spannungsquelle 71, eine zweite Spannungsquelle 72 und eine Gradientenspuleneinheit 19 auf. Das Schaltbild umfasst zwei Schaltkreise, wobei die erste Spannungsquelle 71 und die zweite Spannungsquelle 72 voneinander verschiedenen Schaltkreisen zugeordnet sind.

Der erste Schaltkreis umfasst die erste Spannungsquelle 71, eine erste Primärspule 40 mit einer ersten Primärspulenwicklung 41 und einer zweiten Primärspulenwicklung 42, und eine erste Sekundärspule 50 mit einer ersten Sekundärspulenwicklung 51. Dabei ist die erste Sekundärspulenwicklung 51 mit der ersten Spannungsquelle 71 und der ersten Primärspule 40 in Serie geschaltet. Die erste Primärspulenwicklung 41 und die zweite Primärspulenwicklung 42 sind parallel zur ersten Spannungsquelle 71 geschaltet. Sofern die erste Spannungsquelle 71 einen ersten Strom induziert, sind die erste Primärspulenwicklung 41 und die zweite Primärspulenwicklung 42 gemeinsam dazu ausgebildet, einen Magnetfeldgradient in eine erste Richtung zu erzeugen. Die erste Sekundärspulenwicklung 51 erzeugt in diesem Fall aufgrund des ersten Stromes ein magnetisches Gegenfeld, welches eine Abschirmung des Magnetfeldgradienten in die erste Richtung insbesondere außerhalb der Gradientenspuleneinheit 19 bewirkt.

Der zweite Schaltkreis umfasst die zweite Spannungsquelle 72, eine zweite Primärspule 45 mit einer dritten Primärspulenwicklung 43 und eine zweite Sekundärspule 52 mit einer zweiten Sekundärspulenwicklung 53. Dabei ist die zweite Sekundärspulenwicklung 53 mit der zweiten Spannungsquelle 72 und der zweiten Primärspule 45, also auch der dritten Primärspulenwicklung 43, in Serie geschaltet. Sofern die zweie Spannungsquelle 71 einen zweiten Strom induziert, ist die dritte Primärspulenwicklung 43 dazu ausgebildet, einen Magnetfeldgradient in eine zweite Richtung zu erzeugen. Die zweite Sekundärspulenwicklung 53 erzeugt in diesem Fall aufgrund des zweiten Stromes ein magnetisches Gegenfeld, welches eine Abschirmung des Magnetfeldgradienten in die zweite Richtung insbesondere außerhalb der Gradientenspuleneinheit 19 bewirkt.

Bei Vorhandensein des ersten Stromes beträgt in der ersten Primärspulenwicklung und/oder der zweiten Primärspulenwicklung eine Stromdichte maximal 70%, bevorzugt maximal 60%, besonders bevorzugt maximal 55% einer Stromdichte in der dritten Primärspulenwicklung 43 bei Vorhandensein des zweiten Stromes. Für diesen Zusammenhang entspricht der erste Strom am Ausgang der ersten Spannungsquelle 71 vorzugsweise dem zweiten Strom am Ausgang der zweiten Spannungsquelle 72. Im Allgemeinen ist eine zeitliche Korrelation einer Ausgabe des ersten Stromes durch die erste Spannungsquelle 71 mit einer Ausgabe des zweiten Stromes durch die zweite Spannungsquelle 72 durch die auszuspielende MR-Steuerungssequenz bestimmt. Die auszuspielende MR-Steuerungssequenz bestimmt typischerweise auch die Stärke des ersten Stromes und des zweiten Stromes, welche Stärke im Allgemeinen zeitlich variiert.

Das Gradientensystem kann beispielsweise für die folgenden Werte ausgelegt sein: Der erste Strom am Ausgang der ersten Spannungsquelle 71 beträgt 500 A. Die erste Primärspule ist dazu ausgelegt, einen Magnetfeldgradient in die erste Richtung mit einer Sensitivität von 100 µT/A/m zu erzeugen. Der ohmsche Widerstand der ersten Primärspule 40 beträgt 50 mΩ, da sich der erste Strom auf die erste Primärspulenwicklung 41 und die zweite Primärspulenwicklung 42 aufteilt. Der ohmsche Widerstand der ersten Sekundärspule 50 beträgt 50 mΩ, da die erste Sekundärspule 50 die halbe Windungszahl der entsprechenden ersten Primärspule 40 aufweist. Der ersten Primärspule 40 sind zwei Kühlungslagen 61, 62 zugeordnet.

Im Vergleich dazu kann ein herkömmliches Gradientensystem, oder beispielsweise auch der zweite Schaltkreis umfassend die zweite Spannungsquelle 72, die zweite Primärspule 45 und die zweite Sekundärspule 52 wie folgt ausgelegt sein: Der zweite Strom am Ausgang der zweiten Spannungsquelle 72 beträgt 500 A. Die erste Primärspule ist dazu ausgelegt, einen Magnetfeldgradient in die erste Richtung mit einer Sensitivität von 100 µT/A/m zu erzeugen. Der ohmsche Widerstand der zweiten Primärspule 45 beträgt 100 mΩ, da der zweite Strom nur an die dritte Primärspulenwicklung 43 abgegeben werden kann und damit die Stromdichte doppelt so groß ist wie in der ersten Primärspulenwicklung 41 oder zweiten Primärspulenwicklung 42. Der ohmsche Widerstand der zweiten Sekundärspule 52 beträgt 50 mΩ, da die zweite Sekundärspule 52 die halbe Windungszahl der entsprechenden zweiten Primärspule 45 aufweist. Der zweiten Primärspule 45 ist nur eine Kühlungslage, die dritte Kühlungslage 63, zugeordnet.

Der zweite Schaltkreis weist demnach im Vergleich zum ersten Schaltkreis umfassend die erste Spannungsquelle 71, die erste Primärspule 40 und die erste Sekundärspule 50 einen um etwa 50% größeren ohmschen Gesamtwiderstand auf und eine um 50% reduzierte Kühlkapazität für die Primärspule. Dadurch kann im ersten Schaltkreis die nominale Amplitude des Magnetfeldgradienten in die erste Richtung im Vergleich zur nominalen Amplitude des Magnetfeldgradienten in die zweite Richtung um 25% erhöht werden, wobei die erste Spannungsquelle 71 beibehalten werden kann. Die Leistungsgrenze des ersten Schaltkreises wird typischerweise durch die erste Spannungsquelle 71 und/oder einen von der ersten Spannungsquelle 71 umfassten Verstärker bestimmt. Dabei kann ein Maximalstrom einer Transistorbank, beispielsweise 25 kW, oder eine maximale Leistung eines Transformators, beispielsweise 38 kW, begrenzend wirken.

Wird die erste Spannungsquelle 71 der verbesserten Kühlkapazität angepasst, so ist aufgrund des um 33% reduzierten ohmschen Widerstandes des ersten Schaltkreises ein $\sqrt{1{,}5}$ mal höherer Strom möglich. Die Verdopplung der Kühlkapazität ermöglicht einen $\sqrt{2}$ mal höheren Strom. Insgesamt kann dadurch die Amplitude des Magnetfeldgradienten in die erste Richtung 1,7 mal so groß gewählt werden wie die Amplitude des Magnetfeldgradienten in die zweite Richtung.

Figur 5 zeigt ein Schaltbild eines nicht-erfindungsgemässen Gradientensystems 30. Es unterscheidet sich von dem in Figur 4 dargestellten Schaltbild in der Ausführungsform der ersten Primärspule 40. Die erste Primärspule 40 umfasst die erste Primärspulenwicklung 41 und die zweite Primärspulenwicklung 42, welche mit der ersten Spannungsquelle 71 in Serie geschalten sind. Ebenso ist die erste Sekundärspule 50 mit der ersten Spannungsquelle 71

in Serie geschalten.

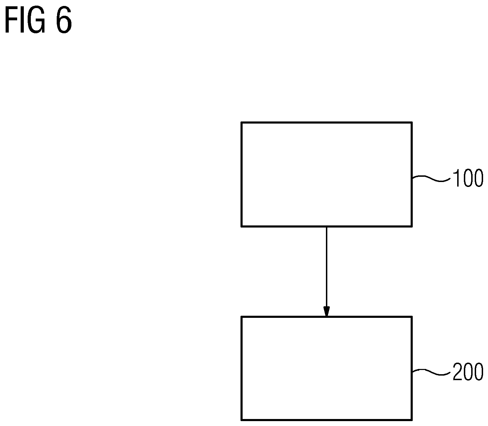

Figur 6 zeigt ein Ablaufdiagramm eines erfindungsgemäßen Verfahrens zu einer Auslegung einer ersten Primärspulenwicklung 41 und einer zweiten Primärspulenwicklung 42 einer ersten Primärspule 40 einer Gradientenspuleneinheit 19. Die Gradientenspuleneinheit 19 umfasst die erste Primärspule 40 und eine erste Sekundärspule 50, wobei die erste Primärspulenwicklung 41 und die zweite Primärspulenwicklung 42 parallel mit einer ersten Spannungsquelle 71 verschaltet sind. Das erfindungsgemäße Verfahren startet mit Verfahrensschritt 100, gemäß dem ein Magnetfeldgradient in eine erste Richtung vorgegeben wird. Anschließend wird in Verfahrensschritt 200 eine Geometrie und/oder eine elektrische Eigenschaft der ersten Primärspulenwicklung 41 und der zweiten Primärspulenwicklung 42 derart bestimmt, dass bei Induktion eines ersten Stromes durch die erste Spannungsquelle 71 gemeinsam von der ersten Primärspulenwicklung 41 und der zweiten Primärspulenwicklung 42 der Magnetfeldgradient in die erste Richtung erzeugt wird.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

1.   Gradientensystem (30) umfassend eine erste Spannungsquelle (71) und eine Gradientenspuleneinheit (19) umfassend eine erste Primärspule (40) und eine erste Sekundärspule (50), wobei die erste Primärspule (40) eine erste Primärspulenwicklung (41) und eine zweite Primärspulenwicklung (42) umfasst, welche erste Primärspulenwicklung (41) und welche zweite Primärspulenwicklung (42)

   - jeweils auf einer Mantelfläche eines Kreiszylinders angeordnet und zueinander radial beabstandet sind,
   - mit der ersten Spannungsquelle (71) verschaltet sind, und
   - gemeinsam dazu ausgebildet sind, einen Magnetfeldgradienten in eine erste Richtung zu erzeugen, sofern die erste Spannungsquelle (71) einen ersten Strom induziert,

   wobei die erste Primärspulenwicklung (41) und die zweite Primärspulenwicklung (42) parallel zu der ersten Spannungsquelle (71) geschaltet sind.

2.   Gradientensystem nach Anspruch 1, wobei die Gradientenspuleneinheit eine der ersten Primärspulenwicklung zugeordnete erste Kühlungslage und eine der zweiten Primärspulenwicklung zugeordnete zweite Kühlungslage umfasst.

3.   Gradientensystem (30) nach einem der vorangehenden Ansprüche, wobei die Gradientenspuleneinheit (19) eine zweite Primärspule (45) mit einer dritten Primärspulenwicklung (43) umfasst, welche dritte Primärspulenwicklung (43) mit einer zweiten Spannungsquelle (72) in Serie geschaltet ist und dazu ausgebildet ist, einen Magnetfeldgradient in eine zweite Richtung zu erzeugen, sofern die zweite Spannungsquelle (72) einen zweiten Strom induziert.

4.   Gradientensystem (30) nach Anspruch 3, wobei bei Vorhandensein des ersten Stromes eine Stromdichte in der ersten Primärspulenwicklung (41) und/oder der zweiten Primärspulenwicklung (42) maximal 70% einer Stromdichte in der dritten Primärspulenwicklung (43) bei Vorhandensein des zweiten Stromes beträgt.

5.   Gradientensystem (30) nach einem der Ansprüche 3 bis 4, wobei die Gradientenspuleneinheit (19) eine der dritten Primärspulenwicklung (43) zugeordnete dritte Kühlungslage (63) umfasst.

6.   Gradientensystem (30) nach einem der vorangehenden Ansprüche, wobei die erste Sekundärspule (50) eine erste Sekundärspulenwicklung (51) umfasst, welche erste Sekundärspulenwicklung (51) mit der ersten Spannungsquelle (71) in Serie geschaltet ist und eine Abschirmung des Magnetfeldgradienten in die erste Richtung bewirkt, sofern die erste Spannungsquelle (71) den ersten Strom induziert.

7.   Magnetresonanzgerät (11) umfassend ein Gradientensystem (30) nach einem der vorangehenden Ansprüche, einen Hauptmagneten (17) und eine Hochfrequenzantenneneinheit (20).

8.   Magnetresonanzgerät nach Anspruch 7, wobei ein definierter hohlzylindrischer Abstandsbereich zwischen dem Hauptmagnet und der Hochfrequenzantenneneinheit die Gradientenspuleneinheit (19) mit der ersten Primärspule (40) umfassend die erste Primärspulenwicklung (41) und die zweite Primärspulenwicklung (42) umfasst.

9.   Verfahren zu einer Auslegung einer ersten Primärspulenwicklung (41) und einer zweiten Primärspulenwicklung (42) einer ersten Primärspule (40) einer Gradientenspuleneinheit (19) eines Gradientensystems (30), welches eine erste Spannungsquelle (71) umfasst, wobei die erste Primärspulenwicklung (41) und die zweite Primärspulenwicklung (42), jeweils angeordnet auf einer Mantelfläche eines Kreiszylinders und zueinander radial beabstandet, parallel mit der ersten Spannungsquelle (71) verschaltet sind, umfassend die folgenden Verfahrensschritte:

- Vorgabe eines Magnetfeldgradienten in eine erste Richtung
- Bestimmung einer Geometrie und/oder einer elektrischen Eigenschaft der ersten Primärspulenwicklung (41) und der zweiten Primärspulenwicklung (42) derart, dass bei Induktion eines ersten Stromes durch die erste Spannungsquelle (71) gemeinsam von der ersten Primärspulenwicklung (41) und der zweiten Primärspulenwicklung (42) der Magnetfeldgradient in die erste Richtung erzeugt wird.

## Claims

1. Gradient system (30) comprising a first voltage source (71) and a gradient coil unit (19) comprising a first primary coil (40) and a first secondary coil (50), wherein the first primary coil (40) comprises a first primary coil winding (41) and a second primary coil winding (42), which first primary coil winding (41) and which second primary coil winding (42)

   - are in each case arranged on a peripheral surface of a circular cylinder and are radially at a distance from one another,
   - are electrically connected to the first voltage source (71), and
   - are jointly embodied to generate a magnetic field gradient in a first direction provided that the first voltage source (71) induces a first current,

   wherein the first primary coil winding (41) and the second primary coil winding (42) are connected to the first voltage source (71) in parallel.

2. Gradient system according to claim 1, wherein the gradient coil unit comprises a first cooling layer assigned to the first primary coil winding and a second cooling layer assigned to the second primary coil winding.

3. Gradient system (30) according to one of the preceding claims, wherein the gradient coil unit (19) comprises a second primary coil (45) with a third primary coil winding (43), wherein the third primary coil winding (43) is connected to a second voltage source (72) in series and is embodied to generate a magnetic field gradient in a second direction provided that the second voltage source (72) induces a second current.

4. Gradient system (30) according to claim 3, wherein, in the presence of the first current, a current density in the first primary coil winding (41) and/or the second primary coil winding (42) is maximum 70% of a current density in the third primary coil winding (43) in the presence of the second current.

5. Gradient system (30) according to one of claims 3 to 4, wherein the gradient coil unit (19) comprises a third cooling layer (63) assigned to the third primary coil winding (43).

6. Gradient system (30) according to one of the preceding claims, wherein the first secondary coil (50) comprises a first secondary coil winding (51), which first secondary coil winding (51) is connected to the first voltage source (71) in series and effects screening of the magnetic field gradient in the first direction provided that the first voltage source (71) induces the first current.

7. Magnetic resonance device (11) comprising a gradient system (30) according to one of the preceding claims, a main magnet (17) and a radio-frequency antenna unit (20).

8. Magnetic resonance device according to claim 7, wherein a defined hollow cylindrical distance range between the main magnet and the radio-frequency antenna unit contains the gradient coil unit (19) with the first primary coil (40) comprising the first primary coil winding (41) and the second primary coil winding (42).

9. Method for designing a first primary coil winding (41) and a second primary coil winding (42) of a first primary coil (40) of a gradient coil unit (19) of a gradient system (30) which comprises a first voltage source (71), wherein the first primary coil winding (41) and the second primary coil winding (42), in each case arranged on a peripheral surface of a circular cylinder and radially at a distance from one another, are electrically connected to the first voltage source (71) in parallel, comprising the following method steps:

   - specification of a magnetic field gradient in a first direction
   - determination of a geometry and/or an electrical property of the first primary coil winding (41) and the second primary coil winding (42) such that, on the induction of a first current through the first voltage source (71), the magnetic field gradient is generated in the first direction jointly by the first primary coil winding (41) and the second primary coil winding (42).

## Revendications

1. Système (30) à gradient comprenant une première source (71) de tension et une unité (19) de bobines à gradient comprenant une première bobine (40) primaire et une première bobine (50) secondaire, dans lequel la première bobine (40) primaire comprend un premier enroulement (41) de bobine primaire et un deuxième enroulement (42) de bobine primaire, lequel premier enroulement (41) de bobine primaire et lequel deuxième enroulement (42) de bobine primaire

   - sont disposés respectivement sur une surface latérale d'un cylindre de section transversale circulaire et sont à distance radialement l'un de l'autre,
   - sont connectés à la première source (71) de tension, et
   - sont constitués conjointement pour produire un gradient de champ magnétique dans une première direction, pour autant que la première source (71) de tension induise un premier courant,

   dans lequel le premier enroulement (41) de la bobine primaire et le deuxième enroulement (42) de la bobine primaire sont montés en parallèle par rapport à la première source (71) de tension.

2. Système à gradient suivant la revendication 1, dans lequel l'unité de bobine à gradient comprend une première couche de refroidissement associée au premier enroulement de la bobine primaire et une deuxième couche de refroidissement associée au deuxième enroulement de la bobine primaire.

3. Système (30) à gradient suivant l'une des revendications précédentes, dans lequel l'unité (19) de bobine à gradient comprend une deuxième bobine (45) primaire ayant un troisième enroulement (43) de bobine primaire, lequel troisième enroulement (43) de bobine primaire est monté en série avec une deuxième source (72) de tension et est constitué pour produire un gradient de champ magnétique dans une deuxième direction, pour autant que la deuxième source (72) de tension induise un deuxième courant.

4. Système (30) à gradient suivant la revendication 3, dans lequel, en présence du premier courant, une densité de courant dans le premier enroulement (41) de la bobine primaire et/ou dans le deuxième enroulement (42) de la bobine primaire représente au maximum 70% d'une densité de courant dans le troisième enroulement (43) de la bobine primaire, en présence du deuxième courant.

5. Système (30) à gradient suivant l'une des revendications 3 à 4, dans lequel l'unité (19) de bobine à gradient comprend une troisième couche (63) de refroidissement associée au troisième enroulement (43) de la bobine primaire.

6. Système (30) à gradient suivant l'une des revendications précédentes, dans lequel la première bobine (50) secondaire comprend un premier enroulement (51) de bobine secondaire, lequel premier enroulement (51) de la bobine secondaire est monté en série avec la première source (71) de tension et provoque un blindage du gradient de champ magnétique dans la première direction, pour autant que la première source (71) de tension induise le premier courant.

7. Appareil (11) à résonnance magnétique comprenant un système (30) à gradient suivant l'une des revendications précédentes, un aimant (17) principal et une unité (20) d'antenne à haute fréquence.

8. Appareil à résonnance magnétique suivant la revendication 7, dans lequel une zone de mise à distance cylindrique, creuse, définie entre l'aimant principal et l'unité d'antenne à haute fréquence comprend l'unité (19) de bobine à gradient ayant la première bobine (40) primaire comprenant le premier enroulement (41) de la bobine primaire et le deuxième enroulement (42) de la bobine primaire.

9. Procédé de conception d'un premier enroulement (41) de bobine primaire et d'un deuxième enroulement (42) de bobine primaire d'une première bobine (40) primaire d'une unité (19) de bobine à gradient d'un système (30) à gradient, lequel comprend une première source (71) de tension, dans lequel le premier enroulement (41) de la bobine primaire et le deuxième enroulement (42) de la bobine primaire, disposés chacun sur une surface latérale d'un cylindre de section transversale circulaire et à distance radialement l'un de l'autre, sont montés en parallèle avec la première source (71) de tension, comprenant les stades de procédé suivant :

   - prescription d'un gradient de champ magnétique dans une première direction
   - détermination d'une géométrie et/ou d'une propriété électrique du premier enroulement (41) de la bobine primaire et du deuxième enroulement (42) de la bobine primaire, de manière à produire le gradient de champ magnétique dans la première direction à l'induction

d'un premier courant dans la première source (71) de tension conjointement par le premier enroulement (41) de la bobine primaire et le deuxième enroulement (42) de la bobine primaire.

FIG 1

# FIG 2

17

154 — 155
166
152 — 153
165
150 — 151
164

119 — 180

144 — 145
163
142 — 143
162
140 — 141
161

20

(Stand der Technik)

# FIG 3

FIG 4

FIG 5

FIG 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102013208631 B3
- DE 102011083204 A1
- US 2005179434 A1
- US 2003197507 A1
- EP 0587423 A1
- US 2016091576 A1
- JP 2011010760 A
- US 5334937 A